# EUROPEAN PATENT APPLICATION

(11) **EP 2 738 748 A1**
(43) Date of publication of application: **04.06.2014**
(21) Application number: 12768040.3
(22) Date of filing: 21.04.2012
(51) Int. Cl.: G08B 21/20, G06K 7/00, G06K 19/07, A61F 13/42

(54) **WIRELESS BEDWETTING ALARM AND DISPOSABLE DIAPER PRODUCT**

(30) Priority: 29.07.2011 CN 201110215759
(71) Applicant: Advance Technology Limited, Shenzhen, Guangdong 518000 (CN)
(72) Inventor: LI, Yaolin, Shenzhen, Guangdong 518000 (CN)
(74) Representative: ProI European Patent Attorneys
(86) International application number: PCT/CN2012/074492
(87) International publication number: WO 2012/136157

(57) **Abstract**

The present invention claims a wireless bedwetting alarm and a disposable diaper product comprising the alarm, wherein the alarm comprises a sensor component, an RFID reader, and an alarm terminal; the sensor component is a passive RFID tag or a moisture sensor; the sensor obviates the need for a standalone power source apparatus for power supply; power is supplied by receiving and converting into electricity the energy wirelessly emitted by the RFID reader, or by obtaining body energy, temperature, or movement of the body of a user; the RFID reader uses a wireless mode for transmitting a signal to the alarm terminal to raise an alarm. The present invention adopts passive technology, obviates the use of battery, and is convenient to use, disposable, harmless to human, and of low costs.

## Description

### FIELD OF THE INVENTION

The present invention relates to a bedwetting alarm, specifically to the bedwetting alarm with wireless signal transmission.

### BACKGROUND OF THE INVENTION

There are many kinds of bedwetting alarms on the market. There are two major working principles:

The first kind is to connect the moister sensor with the alarm through the signal wire. When the moister sensor detects bedwetting, it sends a signal to the alarm about the bedwetting, and then the alarm raises an alarm and vibrates to notify the caregivers.

The second kind is to change wired signal transmission of the first kind into wireless transmission. The detecting terminal of the sensor comprises a sensor, a controlling IC, a power supply and a wireless transmitting module, being responsible for converting the detected bedwetting signal into electric signal and sending it to the receiving terminal by the wireless transmitting module with certain coding; and the receiving terminal comprises a controlling IC, a power supply, an alarm and a wireless receiving module.

The first kind adopts wired transmission, so there is distance limit between the alarm and the caregiver and the alarm is close to the user; as a result, the sound of alarm would wake the user (generally kids or the old people).

The second kind adopts wireless transmission, so it makes some improvement against the distance limit, but the detecting terminal of the sensor adopts separate battery for power supply, which is not convenient for use. Once the battery is dead, the sensor cannot be used; besides, the battery has certain weight and makes the price higher than those without battery.

Further, the above two are not disposable.

### SUMMARY OF THE INVENTION

In order to resolve the problem of the existing technology, the present invention discloses a wireless bedwetting alarm, which senses bedwetting using the passive RFID tag or the moister sensor, and then sends the bedwetting signal through wireless methods (RF/Bluetooth/Z-wave/GSM/CDMA/WiFi/WiMAX) to a handheld terminal alarm taken by the caregiver.

The present invention discloses a wireless bedwetting alarm comprising a sensor component, an RFID reader, and an alarm terminal, wherein the sensor component is a passive RFID tag or a moisture sensor; the sensor obviates the need for a standalone power source apparatus for power supply; power is supplied by receiving and converting into electricity the energy wirelessly emitted by the RFID reader, or by obtaining body energy, temperature, or movement of the body of a user; the RFID reader uses a wireless mode for transmitting a signal to the alarm terminal to raise an alarm.

As a further improvement of the present invention, the RFID reader is coupled with the passive RFID tag, and the magnetic variation that sends signal through the RFID reader is sensed and rectified by the passive RFID tag, thus to generate voltage and drive the integrated circuit of the passive RFID tag, as well as send feedback signal to the RFID reader.

As a further improvement of the present invention, the passive RFID tag or the moisture sensor is a human body energy collector that collects the human body energy. The passive RFID tag or the moisture sensor is equipped with a special equipment (for example, heat recovery system), which can effectively absorb heat radiated from human body and convert the heat energy into electric energy. In addition, human body energy like body temperature, urine, sweat, spit, secreta, walking and other energy that can be obtained from human body, can all be converted into electric energy.

As a further improvement of the present invention, the sensor component generates electricity using temperature difference of human body. By using the Seebeck effect principle, the heat energy can be converted into electric energy through temperature difference. There is the temperature difference between human body and environment, so the heat energy can be converted into electric energy through thermoelectric technology. If two kinds of semiconductors are combined together, and one end thereof is in high temperature state (heat source), while the other end is open-circuited in low temperature state (cold source), open circuit voltage is generated at the cold end, which is called thermo-electromotive force, or Seebeck electromotive force.

As a further improvement of the present invention, the sensor component generates electricity through reaction between magnesium rod or carbon rod and urine.

As a further improvement of the present invention, the passive RFID tag includes Integrated Circuit (IC) inside to save and process data, generates the signal to be sent and analyzes TF signal sent from the reader; besides, the passive RFID tag further comprises an antenna that can receive and send signals.

A disposable diaper product, comprising the wireless bedwetting alarm according to any of claims 1 to 6, and the disposable diaper product is paper nappy or paper diaper

The beneficial effects of the present invention are as follows: the present invention adopts passive technology without need of battery; it is convenient for domestic use and disposable, low in cost and does not cause harm to human body; the whole system is of wireless communication, so there is no distance limit of wire; it adopts the passive RFID tag to detect signal, send signal to the handheld alarm terminal with RFID reader as intermediate station, and notify the caregiver about bedwetting, so the caregiver and user can know the bedwetting anytime anywhere without distance limit and disturb to the wearer.

### BRIEF DESCRIPTION OF THE DRAWINGS

Fig. 1 is the structure diagram of the present invention;
Fig. 2 is the functional block diagram of the present invention;
Fig. 3 is the structural block diagram of hardware of the present invention;
Fig. 4 is the schematic circuit diagram of the RFID tag;
Fig. 5 is the structure diagram of paper nappy with the RFID tag; and
Fig. 6 is the internal structural block diagram of the reader.

### DETAILED DESCRIPTION OF THE PREFERRED EMBODIMENTS

The following is detailed description of the present invention in combination with the drawings and embodiments.

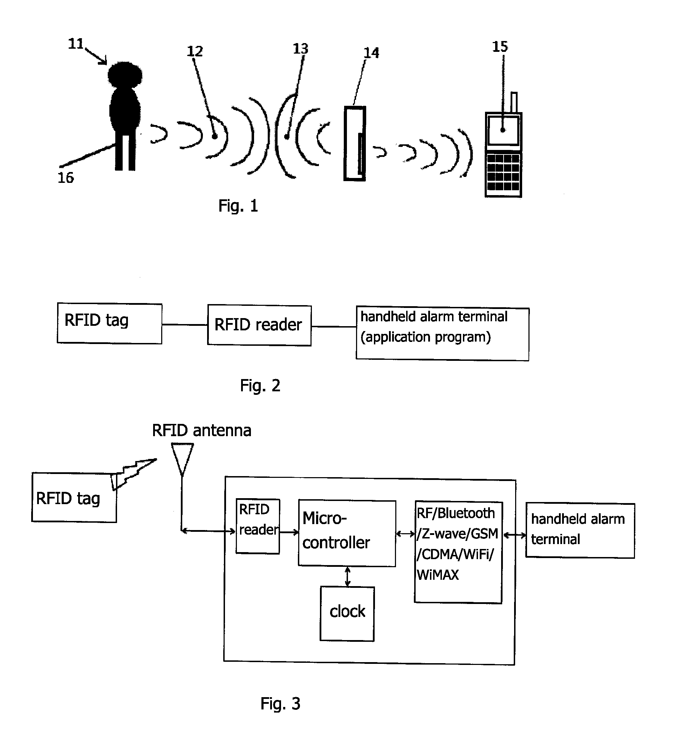

In Fig. 1, 11 is the user, 12 is the passive RFID tag signal, 13 is the signal power provided by the reader, 14 is the reader, 15 is the alarm terminal, and 16 is the bedwetting point and the passive RFID tag.

In Fig. 2, the RFID system comprises the RFID tag, RFID reader and application software. The working principle is that the reader sends radio wave energy of certain frequency to the RFID tag, so as to drive the RFID tag circuit to send out the data in the tag; at this time, the reader receives and reads data in sequence, and sends the data to the application program for relevant processing.

In Fig. 3, the RFID reader is the center to control and process the RFID system information. The reader is generally comprised of micro controller, timer, and transceiver modules like RF/Bluetooth//Z-wave/GSM/CDMA/WiFi/WiMAX unit. The mode 1 is to communicate with the passive RFID tag, and the mode 2 is to communicate with the RF/Bluetooth//Z-wave/GSM/CDMA/WiFi/WiMAX module, thus to send signal to the alarm terminal taken by the caregiver.

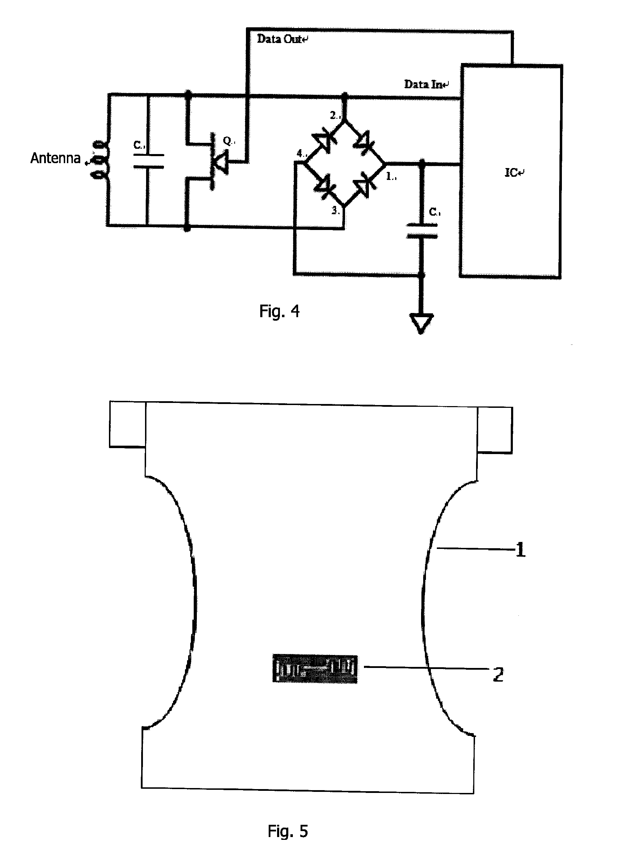

In Fig. 4, the RFID tag is the information carrier of the system, comprising coupling elements (coils, antenna and so on) and IC to be the passive unit.

In Fig. 5, 1 is the paper nappy, and 2 is the passive RFID tag.

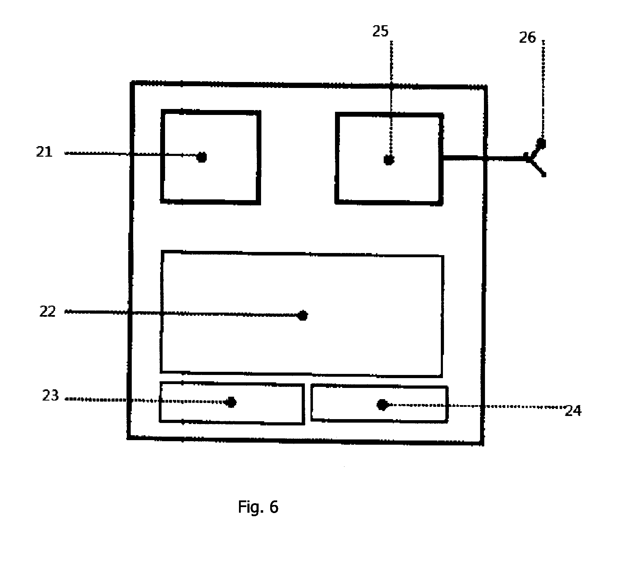

In Fig. 6, 21 is the RFID module, 22 is the controlling chip, 23 is the display interface, 24 is other interface, 25 is the RF/Bluetooth//Z-wave/GSM/CDMA/WiFi/WiMAX module, and 26 is the antenna.

RF: Radio Frequency

Z-Wave is a new low-cost low-power short-range wireless communication technology that is based on radio frequency, highly reliable, and applicable for network.

GSM: Global System of Mobile communication

CDMA: Code Division Multiple Access, which is a technology used for radio communication.

Wi-Fi is a technology that enables terminals like personal computers and handheld devices (for example, PDA and mobile phone) to be mutually connected with each other in wireless way.

WiMax: Worldwide Interoperability for Microwave Access

The technical solution of the present invention is realized as follows:

### 1) Components:

a) Sensing part: passive RFID tag or moister sensor, which obviates the use of separate power unit for power supply:
- It can receive the energy emitted by the RFID reader wirelessly and convert into electricity. The reader and the tag have coupling action like transformer, and the magnetic variation that sends signal through the RFID reader is sensed and rectified by the passive RFID tag, thus to generate voltage and drive the integrated circuit of the passive RFID tag, as well as send feedback signal to the RFID reader.
- Or it can receive human body energy, temperature or movement from the user. The human body energy includes body temperature, urine, sweat, spit, secreta, walking and other energy that can be obtained from human body. The ultrahigh frequency RFID tag that would not be influenced by moister is used. This tag or sensor is like a collector that collects the human body energy. For example, the temperature difference between human and the nature can be used to generate electricity. There is certain difference between human body temperature and the average temperature, by using the Seebeck effect principle, the heat energy can be converted into electric energy. In addition, for liquids like urine, magnesium rod or carbon rod can be used for reaction with water or other chemicals in the liquid to generate electricity. Or just like the clockwork spring of the mechanical clocks and watches, when the spring is tightened, energy is released in constant rate through the ratchet wheel, thus to generate electricity. The RFID tag includes IC inside, which can save and process data, generate signals to be sent and analyzes the RF signal sent from the reader; in addition, the RFID tag further comprises an antenna that can receive and send signals.

b) RFID reader with two working modes: the mode 1 is to communicate with the passive RFID tag, and the mode 2 is to communicate with the RF/Bluetooth//Z-wave/GSM/CDMA/WiFi/WiMAX module, thus to send signal to the alarm terminal (alarm terminal taken by the caregiver). The caregiver carries the alarm terminal, such as mobile phone, which has already been equipped with wireless modules (such as Bluetooth/WiFi). By taking advantage of this, it is only necessary to establish relevant wireless modules in the reader and send data with the communication protocol, thus to realize communication between the two devices through the existing wireless IC.

c) The caregiver carries the alarm terminal (such as alarm, mobile phone or handheld device) with alarm program, and the alarm terminal can raise an alarm while receiving signals.

### 2) Working principle:

### Specific instructions:

The sensing circuit (as shown in Fig.5) is placed on the user, namely in the paper nappy, or printed on the paper nappy/underpants using printing technology, or printed on film using printing technology and sewed on the paper nappy/underpants. The RFID reader is placed about 1 meter away from the user (There is no connection between the sensing circuit and the RFID reader, so it is very convenient).

Bedwetting determination method 1: communication with the RFID reader is interrupted

(A) in normal situation, namely, if there is no bedwetting, the communication between the sensing circuit and the RFID reader is in normal state. The passive RFID tag periodically transmits effective signals to the RFID reader through the signal power supply provided by the RFID reader.

In actual use, once there is bedwetting, the passive RFID tag becomes disabled in liquid, so it cannot send effective signal to the RFID reader, which means that the communication is interrupted. If the RFID reader cannot receive signal for long time, it means that there is bedwetting. At this time, mode 2 of the RFID reader is activated, namely, thus to send signal to the alarm terminal through RF/Bluetooth//Z-wave/GSM/CDMA/WiFi/WiMAX module to notify the bedwetting.

(B) in normal situation, namely, if there is no bedwetting, the communication between the sensing circuit and the RFID reader is in normal state. The passive RFID tag, by using the human body energy (such as body temperature, urine, sweat, spit, secreta, walking and other energy that can be obtained from human body) or without need of any energy, periodically sends or feeds effective signal to the RFID reader.

In actual use, once there is bedwetting, the passive RFID tag becomes disabled in liquid, so it cannot send effective signal to the RFID reader, which means that the communication is interrupted. If the RFID reader cannot receive signal for long time, it means that there is bedwetting. At this time, mode 2 of the RFID reader is activated, namely, thus to send signal to the alarm terminal through RF/Bluetooth//Z-wave/GSM/CDMA/WiFi/WiMAX module to notify the bedwetting.

### Bedwetting determination method 2: communication between the ultrahigh RFID tag and reader

When there is bedwetting, the ultrahigh RFID tag, which is not affected by moisture, sends signal to the reader using the energy obtained from urine, so as to determine occurrence of bedwetting. At this time, mode 2 of the RFID reader is activated, namely, thus to send signal to the alarm terminal through RF/Bluetooth//Z-wave/GSM/CDMA/WiFi/WiMAX module to notify the bedwetting.

### Bedwetting determination method 3: passive moister sensor

The passive moister sensor can obtain from human body, judge occurrence of bedwetting and notify the RF module. The RF module sends signal to the reader, from which the bedwetting can be determined. At this time, mode 2 of the RFID reader is activated, namely, thus to send signal to the alarm terminal through RF/Bluetooth//Z-wave/GSM/CDMA/WiFi/WiMAX module to notify the bedwetting.

The alarm terminal can be mobile phone or handheld device of the caregiver, and the reader can send short messages to or call the mobile phone of the caregiver; or if bedwetting alarm application program is installed on the mobile phone, when it receives signals sent from the wireless module, it notifies the caregiver.

If the mobile phone is an intelligent mobile phone carrying RFID module, after installing bedwetting alarm application program, it can read the bedwetting signal directly from the tag.

The above content is only detailed description of the present invention in combination with the embodiments, the specific implementation of the present invention is not limited to this. The common technical personnel of this field may further figure out some deduction or replacement within conception of the present invention, but all the deduction or replacement shall fall into the protective scope of the present invention.

## Claims

1. A wireless bedwetting alarm, comprising a sensor component, an RFID reader, and an alarm terminal, wherein the sensor component is a passive RFID tag or a moisture sensor; the sensor obviates the need for a standalone power source apparatus for power supply; power is supplied by receiving and converting into electricity the energy wirelessly emitted by the RFID reader, or by obtaining body energy, temperature, or movement of the body of a user; the RFID reader uses a wireless mode for transmitting a signal to the alarm terminal to raise an alarm.

2. The wireless bedwetting alarm according to claim 1, wherein the RFID reader is coupled with the passive RFID tag, and the magnetic variation that sends signal through the RFID reader is sensed and rectified by the passive RFID tag, thus to generate voltage and drive the integrated circuit of the passive RFID tag, as well as send feedback signal to the RFID reader.

3. The wireless bedwetting alarm according to claim 1, wherein the passive RFID tag or the moisture sensor is a human body energy collector that collects the human body energy.

4. The wireless bedwetting alarm according to claim 1, wherein the sensor component generates electricity using temperature difference of human body.

5. The wireless bedwetting alarm according to claim 1, wherein the sensor component generates electricity through reaction between magnesium rod or carbon rod and urine.

6. The wireless bedwetting alarm according to any of claims 1 to 5, wherein the passive RFID tag includes Integrated Circuit (IC) inside to save and process data, generates the signal to be sent and analyzes TF signal sent from the reader; besides, the passive RFID tag further comprises an antenna that can receive and send signals.

7. A disposable diaper product, comprising the wireless bedwetting alarm according to any of claims 1 to 6.

8. The disposable diaper product according to claim 7, wherein the disposable diaper product is paper nappy or paper diaper.
